# NOUVEAU FASCICULE DE BREVET EUROPEEN

(11) **EP 1 078 074 B2**
(45) Date de publication et mention de la décision concernant l'opposition: **09.10.2013**
(45) Mention de la délivrance du brevet: 02.02.2005
(21) Numéro de dépôt: 99919337.8
(22) Date de dépôt: 17.05.1999
(51) Int. Cl.: C12N 15/56, A23C 9/123, C12N 1/20, C12N 9/38, C12R 1/225

(54) **SOUCHES MUTANTES DE LACTOBACILLUS BULGARICUS DEPOURVUES D'ACTIVITE BETA-GALACTOSIDASE**
MUTANTSTÄMME VON LACTOBACILLUS BULGARICUS FREI VON BETA-GALACTOSIDASE AKTIVITÄT
MUTANT LACTOBACILLUS BULGARICUS STRAINS FREE FROM BETA-GALACTOSIDE ACTIVITY

(30) Priorité: 22.05.1998 FR 9806456
(43) Date de publication de la demande: 28.02.2001
(73) Titulaire: COMPAGNIE GERVAIS DANONE, 75009 Paris (FR)
(72) Inventeur: BENBADIS, Laurent, F-92160 Anthony (FR); BRIGNON, Pierre, F-67200 Strasbourg (FR); GENDRE, François, F-67200 Strasbourg (FR)
(74) Mandataire: Vialle-Presles, Marie José
(86) Numéro de dépôt international: PCT/FR1999/001165
(87) Numéro de publication internationale: WO 1999/061627

(56) Documents cités:
- EP-A- 0 518 096
- WO-A-90/05459

### LISTE DE SEQUENCES

<110> COMPAGNIE GERVAIS DANONE
   BENBADIS, Laurent
   BRIGNON, Pierre
   GENDRE, François
<120> SOUCHES MUTANTES DE LACTOBACILLUS BULGARICUS DEPOURVUES D'ACTIVITE BETA-GALACTOSIDASE
<130> MJPcb191/143
<140>
   <141>
<150> FR9806456
   <151> 1998-05-22
<160> 3
<170> PatentIn Ver. 2.1
<210> 1
   <211> 5059
   <212> ADN
   <213> Lactobacillus bulgaricus
<220>
   <221> CDS
   <222> (122)..(1873)
<220>
   <221> CDS
   <222> (1877)..(4519)
<400> 1
   gcttgtctca cgcttgtcgt acgcggccgg tgcctttggc aacgacgtct tctacgcgac 60
   tctgtcaacc tactttatcg tcttcgtcac cacccacctc tttaatgccg gtgaccacaa 120
   g atg atc ttt atc atc acc aac ttg atc acc gcc atc cgg atc ggg gaa 169
     Met Ile Phe Ile Ile Thr Asn Leu Ile Thr Ala Ile Arg Ile Gly Glu
       1               5                  10                  15
   gtc ctg ctc gac ccc ttg atc ggt aac gcc atc gac cgg acc gaa agc   217
   Val Leu Leu Asp Pro Leu Ile Gly Asn Ala Ile Asp Arg Thr Glu Ser
                20                  25                  30
   cgg tgg ggg aag ttc aag ccc tgg gtt gtg ggc ggg ggg atc atc agc   265
   Arg Trp Gly Lys Phe Lys Pro Trp Val Val Gly Gly Gly Ile Ile Ser
            35                  40                  45
   tca tta gcc ctc tta gcc ctc ttt acc gac ttt ggc ggc att aac caa   313
   Ser Leu Ala Leu Leu Ala Leu Phe Thr Asp Phe Gly Gly Ile Asn Gln
       50                  55                  60
   agc aac ccc gtt gtt tac tta gta atc ttc ggt att gtt tac ttg att   361
   Ser Asn Pro Val Val Tyr Leu Val Ile Phe Gly Ile Val Tyr Leu Ile
    65                  70                  75                  80
   atg gat atc ttc tac tca ttt aaa gac act ggc ttc tgg gcc atg atc   409
   Met Asp Ile Phe Tyr Ser Phe Lys Asp Thr Gly Phe Trp Ala Met Ile
                   85                  90                  95
   ccg gcc ttg tcc ctg gat tcc cgg gaa aga gag aag acc tcc acc ttc    457
   Pro Ala Leu Ser Leu Asp Ser Arg Glu Arg Glu Lys Thr Ser Thr Phe
               100                 105                 110
   gcc aga gtc ggc tcc acc atc ggg gcc aac ctg gtc ggg gta gtc atc    505
   Ala Arg Val Gly Ser Thr Ile Gly Ala Asn Leu Val Gly Val Val Ile
           115                 120                 125
   acc cca atc atc ctc ttc ttc tcg gcc agc aag gcc aac ccc aac ggg    553
   Thr Pro Ile Ile Leu Phe Phe Ser Ala Ser Lys Ala Asn Pro Asn Gly
       130                 135                 140
   gat aag cag ggc tgg ttc ttc ttt gcc ttg atc gtg gcc att gtc ggc    601
   Asp Lys Gln Gly Trp Phe Phe Phe Ala Leu Ile Val Ala Ile Val Gly
   145                 150                 155                 160
   atc ttg acc tca att acc gtt ggt ctt ggt act cac gaa gta aaa tcc    649
   Ile Leu Thr Ser Ile Thr Val Gly Leu Gly Thr His Glu Val Lys Ser
                   165                 170                 175
   gcc ctg cgg gaa agc aat gaa aag acc act ttg aag cag gtc ttt aag    697
   Ala Leu Arg Glu Ser Asn Glu Lys Thr Thr Leu Lys Gln Val Phe Lys
               180                 185                 190
   gtc ctg ggg caa aac gac cag ctc ctc tgg ctg gcc ttt gcc tac tgg    745
   Val Leu Gly Gln Asn Asp Gln Leu Leu Trp Leu Ala Phe Ala Tyr Trp
           195                 200                 205
   ttt tac ggc ctg ggt atc aac acc ctg aac gct ctg caa ctt tac tac    793
   Phe Tyr Gly Leu Gly Ile Asn Thr Leu Asn Ala Leu Gln Leu Tyr Tyr
       210                 215                 220
   ttc tca tac atc tta ggc gat gcc cgc ggc tac agc ctg ctt tac acc    841
   Phe Ser Tyr Ile Leu Gly Asp Ala Arg Gly Tyr Ser Leu Leu Tyr Thr
   225                 230                 235                 240
   atc aac acc ttt gtc ggt tta atc tct gca tcc ttc ttc cca tca ctg    889
   Ile Asn Thr Phe Val Gly Leu Ile Ser Ala Ser Phe Phe Pro Ser Leu
                   245                 250                 255
   gcc aag aag ttc aac aga aat cgc ctc ttc tac gcc tgc atc gcg gtg    937
   Ala Lys Lys Phe Asn Arg Asn Arg Leu Phe Tyr Ala Cys Ile Ala Val
               260                 265                 270
   atg ctg tta ggg atc ggg gtc ttc tcc gtg gcc agc ggt tct ctg gcc    985
   Met Leu Leu Gly Ile Gly Val Phe Ser Val Ala Ser Gly Ser Leu Ala
           275                 280                 285
   ctg tcc ctt gtt ggg gca gaa ttc ttc ttt att ccg cag cct ctg gcc   1033
   Leu Ser Leu Val Gly Ala Glu Phe Phe Phe Ile Pro Gln Pro Leu Ala
       290                 295                 300
   ttc ctg gtc gtt ttg atg atc atc tct gac gct gtt gaa tac ggc cag   1081
   Phe Leu Val Val Leu Met Ile Ile Ser Asp Ala Val Glu Tyr Gly Gln
   305                 310                 315                 320
   ctg aaa act ggc cac aga gac gaa gct ttg acc ctg tct gtc cgg cca   1129
   Leu Lys Thr Gly His Arg Asp Glu Ala Leu Thr Leu Ser Val Arg Pro
   325 330 335
   ttg gtc gat aag ctg ggc ggg gcc ttg tcc aac tgg ttt gtt tcc ttg 1177
   Leu Val Asp Lys Leu Gly Gly Ala Leu Ser Asn Trp Phe Val Ser Leu
   340 345 350
   att gcc tta act gcc ggc atg acc act ggg gcg act gcc tca aca att 1225
   Ile Ala Leu Thr Ala Gly Met Thr Thr Gly Ala Thr Ala Ser Thr Ile
   355 360 365
   aca gct cat ggc cag atg gtc ttc aag tta gct atg ttt gcc tta ccg 1273
   Thr Ala His Gly Gln Met Val Phe Lys Leu Ala Met Phe Ala Leu Pro
   370 375 380
   gca gtc atg ctc ttg atc gct gtt tct att ttc gcc aaa aag gtc ttc 1321
   Ala Val Met Leu Leu Ile Ala Val Ser Ile Phe Ala Lys Lys Val Phe
   385 390 395 400
   ttg act gaa gaa aag cac gcg gaa atc gtc gac cag ctg gaa act caa 1369
   Leu Thr Glu Glu Lys His Ala Glu Ile Val Asp Gln Leu Glu Thr Gln
   405 410 415
   ttc agc caa agc cat gcc caa aag ccg gcg caa gct gaa agc ttc act 1417
   Phe Ser Gln Ser His Ala Gln Lys Pro Ala Gln Ala Glu Ser Phe Thr
   420 425 430
   ttg gcc agc cca gtc tcc gga caa tta atg aac ctg gac atg gtt gac 1465
   Leu Ala Ser Pro Val Ser Gly Gln Leu Met Asn Leu Asp Met Val Asp
   435 440 445
   gac ccg gtc ttt gcc gac aaa aag tta ggc gac ggc ttt gcc ctg gtg 1513
   Asp Pro Val Phe Ala Asp Lys Lys Leu Gly Asp Gly Phe Ala Leu Val
   450 455 460
   cca gca gac ggt aag gtc tac gcg cca ttt gcc ggt act gtc cgc cag 1561
   Pro Ala Asp Gly Lys Val Tyr Ala Pro Phe Ala Gly Thr Val Arg Gln
   465 470 475 480
   ctg gcc aag acc cgg cac tcg atc gtc ctg gaa aat gaa cat ggg gtc 1609
   Leu Ala Lys Thr Arg His Ser Ile Val Leu Glu Asn Glu His Gly Val
   485 490 495
   ttg gtc ttg att cac ctt ggc ctg ggc acg gtc aaa tta aac ggg act 1657
   Leu Val Leu Ile His Leu Gly Leu Gly Thr Val Lys Leu Asn Gly Thr
   500 505 510
   ggc ttt gtc agc tat gtt gaa gag ggc agc cag gta gaa gcc ggc cag 1705
   Gly Phe Val Ser Tyr Val Glu Glu Gly Ser Gln Val Glu Ala Gly Gln
   515 520 525
   cag atc ctg gaa ttc tgg gac ccg gcg atc aag cag gcc aag ctg gac 1753
   Gln Ile Leu Glu Phe Trp Asp Pro Ala Ile Lys Gln Ala Lys Leu Asp
   530 535 540
   gac acg gta atc gtg acc gtc atc aac agc gaa act ttc gca aat agc 1801
   Asp Thr Val Ile Val Thr Val Ile Asn Ser Glu Thr Phe Ala Asn Ser
   545 550 555 560
   cag atg ctc ttg ccg atc ggc cac agc gtc caa gcc ctg gat gat gta 1849
   Gln Met Leu Leu Pro Ile Gly His Ser Val Gln Ala Leu Asp Asp Val
   565 570 575
   ttc aag tta gaa ggg aag aat tag aaa atg agc aat aag tta gta aaa     1897
   Phe Lys Leu Glu Gly Lys Asn         Met Ser Asn Lys Leu Val Lys
               580                     585                 590
   gaa aaa aga gtt gac cag gca gac ttg gcc tgg ctg act gac ccg gaa     1945
   Glu Lys Arg Val Asp Gln Ala Asp Leu Ala Trp Leu Thr Asp Pro Glu
               595                 600                 605
   gtt tac gaa gtc aat aca att ccc ccg cac tcc gac cat gag tcc ttc     1993
   Val Tyr Glu Val Asn Thr Ile Pro Pro His Ser Asp His Glu Ser Phe
           610                 615                 620
   caa agc cag gaa gaa ctg gag gag ggc aag tcc agt tta gtg cag tcc     2041
   Gln Ser Gln Glu Glu Leu Glu Glu Gly Lys Ser Ser Leu Val Gln Ser
       625                 630                 635
   ctg gac ggg gac tgg ctg att gac tac gct gaa aac ggc cag gga cca     2089
   Leu Asp Gly Asp Trp Leu Ile Asp Tyr Ala Glu Asn Gly Gln Gly Pro
   640                 645                 650                 655
   gtc aac ttc tat gca gaa gac ttt gac gat agc aat ttt aag tca gtc     2137
   Val Asn Phe Tyr Ala Glu Asp Phe Asp Asp Ser Asn Phe Lys Ser Val
                   660                 665                 670
   aaa gta ccc ggc aac ctg gaa ctg caa ggc ttt ggc cag ccc cag tat     2185
   Lys Val Pro Gly Asn Leu Glu Leu Gln Gly Phe Gly Gln Pro Gln Tyr
               675                 680                 685
   gtc aac gtc caa tat cca tgg gac ggc agt gag gag att ttc ccg ccc     2233
   Val Asn Val Gln Tyr Pro Trp Asp Gly Ser Glu Glu Ile Phe Pro Pro
           690                 695                 700
   caa att cca agc aaa aat ccg ctc gct tct tat gtc aga tac ttt gac     2281
   Gln Ile Pro Ser Lys Asn Pro Leu Ala Ser Tyr Val Arg Tyr Phe Asp
       705                 710                 715
   ctg gat gaa gct ttc tgg gac aag gaa gtc agc ttg aag ttt gac ggg     2329
   Leu Asp Glu Ala Phe Trp Asp Lys Glu Val Ser Leu Lys Phe Asp Gly
   720                 725                 730                 735
   gcg gca aca gcc atc tat gtc tgg ctg aac ggc cac ttc gtc ggc tac     2377
   Ala Ala Thr Ala Ile Tyr Val Trp Leu Asn Gly His Phe Val Gly Tyr
                   740                 745                 750
   ggg gaa gac tcc ttt acc cca agc gag ttt atg gtt acc aag ttc ctc     2425
   Gly Glu Asp Ser Phe Thr Pro Ser Glu Phe Met Val Thr Lys Phe Leu
               755                 760                 765
   aag aaa gaa aat aac cgc ctg gca gtg gct ctc tac aag tat tct tcc     2473
   Lys Lys Glu Asn Asn Arg Leu Ala Val Ala Leu Tyr Lys Tyr Ser Ser
           770                 775                 780
   gcc tcc tgg ctg gaa gac cag gac ttc tgg cgc atg tct ggt ttg ttc     2521
   Ala Ser Trp Leu Glu Asp Gln Asp Phe Trp Arg Met Ser Gly Leu Phe
       785                 790                 795
   aga tca gtg act ctt cag gcc aag ccg cgt ctg cac ttg gag gac ctt     2569
   Arg Ser Val Thr Leu Gln Ala Lys Pro Arg Leu His Leu Glu Asp Leu
   800                 805                 810                 815
   aag ctt acg gcc agc ttg acc gat aac tac caa aaa gga aag ctg gaa    2617
   Lys Leu Thr Ala Ser Leu Thr Asp Asn Tyr Gln Lys Gly Lys Leu Glu
                   820                 825                 830
   gtc gaa gcc aat att gcc tac cgc ttg cca aat gcc agc ttt aag ctg    2665
   Val Glu Ala Asn Ile Ala Tyr Arg Leu Pro Asn Ala Ser Phe Lys Leu
               835                 840                 845
   gaa gtg cgg gat agt gaa ggt gac ttg gtt gct gaa aag ctg ggc cca    2713
   Glu Val Arg Asp Ser Glu Gly Asp Leu Val Ala Glu Lys Leu Gly Pro
           850                 855                 860
   atc aga agc gag cag ctg gaa ttc act ctg gct gat ttg cca gta gct    2761
   Ile Arg Ser Glu Gln Leu Glu Phe Thr Leu Ala Asp Leu Pro Val Ala
       865                 870                 875
   gcc tgg agc gcg gaa aag cct aac ctt tac cag gtc cgc ctg tat tta    2809
   Ala Trp Ser Ala Glu Lys Pro Asn Leu Tyr Gln Val Arg Leu Tyr Leu
   880                 885                 890                 895
   tac cag gca ggc agc ctc tta gag gtt agc cgg cag gaa gtg ggt ttc    2857
   Tyr Gln Ala Gly Ser Leu Leu Glu Val Ser Arg Gln Glu Val Gly Phe
                   900                 905                 910
   cgc aac ttt gaa cta aaa gac ggg att atg tac ctt aac ggc cag cgg    2905
   Arg Asn Phe Glu Leu Lys Asp Gly Ile Met Tyr Leu Asn Gly Gln Arg
               915                 920                 925
   atc gtc ttc aag ggg gcc aac cgg cac gaa ttt gac agt aag ttg ggc    2953
   Ile Val Phe Lys Gly Ala Asn Arg His Glu Phe Asp Ser Lys Leu Gly
           930                 935                 940
   cgg gct atc aca gaa gag gat atg atc tgg gat atc aag acc atg aag    3001
   Arg Ala Ile Thr Glu Glu Asp Met Ile Trp Asp Ile Lys Thr Met Lys
       945                 950                 955
   cga agc aac atc aat gct gtc cgc tgc tct cac tac ccg aac cag tcc    3049
   Arg Ser Asn Ile Asn Ala Val Arg Cys Ser His Tyr Pro Asn Gln Ser
   960                 965                 970                 975
   ctc ttt tac cgg ctc tgt gac aag tac ggc ctt tac gtc att gat gaa    3097
   Leu Phe Tyr Arg Leu Cys Asp Lys Tyr Gly Leu Tyr Val Ile Asp Glu
                   980                 985                 990
   gct aac ctg gaa agc cac ggc acc tgg gaa aaa gtg ggg ggg cac gaa    3145
   Ala Asn Leu Glu Ser His Gly Thr Trp Glu Lys Val Gly Gly His Glu
               995                 1000                1005
   gat cct agc ttc aat gtt cca ggc gat gac cag cat tgg ctg gga gcc    3193
   Asp Pro Ser Phe Asn Val Pro Gly Asp Asp Gln His Trp Leu Gly Ala
           1010                1015                1020
   agc tta tcc cgg gtg aag aac atg atg gct cgg gac aag aac cat gct    3241
   Ser Leu Ser Arg Val Lys Asn Met Met Ala Arg Asp Lys Asn His Ala
       1025                1030                1035
   tca atc ctg atc tgg tct tta ggc aat gag tct tac gcc ggc act gtc    3289
   Ser Ile Leu Ile Trp Ser Leu Gly Asn Glu Ser Tyr Ala Gly Thr Val
   1040                1045                1050                1055
   ttt gcc caa atg gct gat tac gtc cgg aag gct gat ccg acc cgg gtt    3337
   Phe Ala Gln Met Ala Asp Tyr Val Arg Lys Ala Asp Pro Thr Arg Val
               1060                1065                1070
   cag cac tat gaa ggg gtg acc cac aac cgg aag ttt gac gac gcc acc     3385
   Gln His Tyr Glu Gly Val Thr His Asn Arg Lys Phe Asp Asp Ala Thr
           1075                1080                1085
   cag att gaa agc cgg atg tat gct ccg gcc aag gta att gaa gaa tac     3433
   Gln Ile Glu Ser Arg Met Tyr Ala Pro Ala Lys Val Ile Glu Glu Tyr
       1090                1095                1100
   ttg acc aat aaa cca gcc aag cca ttc atc tca gtt gaa tac gct cac     3481
   Leu Thr Asn Lys Pro Ala Lys Pro Phe Ile Ser Val Glu Tyr Ala His
       1105                1110                1115
   gcc atg ggc aac tcc gtc ggt gac ctg gcc gcc tac acg gcc ctg gaa     3529
   Ala Met Gly Asn Ser Val Gly Asp Leu Ala Ala Tyr Thr Ala Leu Glu
   1120                1125                1130                1135
   aaa tac ccc cac tac cag ggc ggc ttc atc tgg gac tgg att gac caa     3577
   Lys Tyr Pro His Tyr Gln Gly Gly Phe Ile Trp Asp Trp Ile Asp Gln
                   1140                1145                1150
   gga ctg gaa aaa gac ggg cac ctg ctt tat ggg ggc gac ttc gat gac     3625
   Gly Leu Glu Lys Asp Gly His Leu Leu Tyr Gly Gly Asp Phe Asp Asp
               1155                1160                1165
   cgg cca acc gac tat gaa ttc tgc ggg aac ggc ctg gtc ttt gct gac     3673
   Arg Pro Thr Asp Tyr Glu Phe Cys Gly Asn Gly Leu Val Phe Ala Asp
           1170                1175                1180
   cgg act gaa tcg ccg aaa ctg gct aat gtc aag gcc ctt tac gcc aac     3721
   Arg Thr Glu Ser Pro Lys Leu Ala Asn Val Lys Ala Leu Tyr Ala Asn
       1185                1190                1195
   ctt aag tta gaa gta aaa gat ggg cag ctc ttc ctc aaa aac gac aat     3769
   Leu Lys Leu Glu Val Lys Asp Gly Gln Leu Phe Leu Lys Asn Asp Asn
   1200                1205                1210                1215
   tta ttt acc aac agc tca tct tac tac ttc ttg act agt ctt ttg gtc     3817
   Leu Phe Thr Asn Ser Ser Ser Tyr Tyr Phe Leu Thr Ser Leu Leu Val
                   1220                1225                1230
   gat ggc aag ttg acc tac cag agc cgg cct ctg acc ttt ggc ctg gag     3865
   Asp Gly Lys Leu Thr Tyr Gln Ser Arg Pro Leu Thr Phe Gly Leu Glu
               1235                1240                1245
   cct ggc gaa tcc ggg acc ttt gcc ctg cct tgg ccg gaa gtc gct gat     3913
   Pro Gly Glu Ser Gly Thr Phe Ala Leu Pro Trp Pro Glu Val Ala Asp
           1250                1255                1260
   gaa aaa gga gag gtc gtc tac cgg gta acg gcc cac tta aaa gaa gac     3961
   Glu Lys Gly Glu Val Val Tyr Arg Val Thr Ala His Leu Lys Glu Asp
       1265                1270                1275
   ttg cct tgg gcg gat gag ggc ttc act gtg gct gaa gca gaa gaa gta     4009
   Leu Pro Trp Ala Asp Glu Gly Phe Thr Val Ala Glu Ala Glu Glu Val
   1280                1285                1290                1295
   gct caa aag ctg ccg gaa ttt aag ccg gaa ggg cgg cca gat tta gtc     4057
   Ala Gln Lys Leu Pro Glu Phe Lys Pro Glu Gly Arg Pro Asp Leu Val
                       1300                1305                1310
   gat tcc gac tac aac cta ggc ctg aaa gga aat aac ttc caa att ctc    4105
   Asp Ser Asp Tyr Asn Leu Gly Leu Lys Gly Asn Asn Phe Gln Ile Leu
               1315                1320                1325
   ttc tcc aag gtc aag ggc tgg ccg gtt tcc ctc aag tat gcc ggt agg    4153
   Phe Ser Lys Val Lys Gly Trp Pro Val Ser Leu Lys Tyr Ala Gly Arg
           1330                1335                1340
   gaa tac ttg aag cgg ctg ccg gaa ttt acc ttc tgg cgg gcc ctg acg    4201
   Glu Tyr Leu Lys Arg Leu Pro Glu Phe Thr Phe Trp Arg Ala Leu Thr
       1345                1350                1355
   gac aac gac cgg gga gct ggt tac ggc tat gat ctg gcc cgg tgg gaa    4249
   Asp Asn Asp Arg Gly Ala Gly Tyr Gly Tyr Asp Leu Ala Arg Trp Glu
   1360                1365                1370                1375
   aat gcc ggc aag tat gcc cgc ttg aaa gac atc agc tgc gag gtc aag    4297
   Asn Ala Gly Lys Tyr Ala Arg Leu Lys Asp Ile Ser Cys Glu Val Lys
                   1380                1385                1390
   gaa gac tcc gtt ttg gtc aag act gcc ttt acg ttg cct gtc gcc tta    4345
   Glu Asp Ser Val Leu Val Lys Thr Ala Phe Thr Leu Pro Val Ala Leu
               1395                1400                1405
   aag ggt gat tta act gtg acc tat gaa gtc gat gga cgg ggc aag att    4393
   Lys Gly Asp Leu Thr Val Thr Tyr Glu Val Asp Gly Arg Gly Lys Ile
           1410                1415                1420
   gct gta aca gct gac ttc cca ggc gcg gaa gaa gcc ggt ctc ttg cca    4441
   Ala Val Thr Ala Asp Phe Pro Gly Ala Glu Glu Ala Gly Leu Leu Pro
       1425                1430                1435
   gcc ttt ggc ttg aac ctg gcc ctg cca aaa gaa ctg acc gat tac cgc    4489
   Ala Phe Gly Leu Asn Leu Ala Leu Pro Lys Glu Leu Thr Asp Tyr Arg
   1440                1445                1450                1455
   tac tat ggt ctg gga cct aat gag agc taa ccagaccgct tggaaggtaa      4539
   Tyr Tyr Gly Leu Gly Pro Asn Glu Ser
                   1460                1465
   ttacctgggc atctaccagg gagcggtaaa aaagaacttt agcccatacc tgcgtccgca 4599
   ggaaacgggc aaccggagca aggttcgctg gtaccagctc tttgatgaaa agggcggctt 4659
   ggaatttacg gccaatgggg cagacttgaa cttgtctgct ttgccatatt ctgccgccca 4719
   aattgaagca gcggaccacg cttttgaact gactaacaat tacacttggg ttagagcctt 4779
   aagcgcccag atgggggtcg gcggggatga ctcctggggg cagaaggtcc acccggaatt 4839
   ctgcctggat gctcaaaaag cccgccagct ccgcctggtg attcagcccc ttttactaaa 4899
   ataaatgcta caattgactt aacaggatga aattttagta aaagcaaagc gagtgaggaa 4959
   gatggcaacg atcagagaag tgccaaggca gccggcgtgt cgctagcgac ggtttcccgc 5019
   gtcttgaact atgaccagac cctgtcagtc aatgaggcaa                      5059
<210> 2
   <211> 583
   <212> PRT
   <213> Lactobacillus bulgaricus
<400> 2
   Met Ile Phe Ile Ile Thr Asn Leu Ile Thr Ala Ile Arg Ile Gly Glu
   1               5                   10                  15
   Val Leu Leu Asp Pro Leu Ile Gly Asn Ala Ile Asp Arg Thr Glu Ser
               20                  25                  30
   Arg Trp Gly Lys Phe Lys Pro Trp Val Val Gly Gly Gly Ile Ile Ser
           35                  40                  45
   Ser Leu Ala Leu Leu Ala Leu Phe Thr Asp Phe Gly Gly Ile Asn Gln
       50                  55                  60
   Ser Asn Pro Val Val Tyr Leu Val Ile Phe Gly Ile Val Tyr Leu Ile
   65                  70                  75                  80
   Met Asp Ile Phe Tyr Ser Phe Lys Asp Thr Gly Phe Trp Ala Met Ile
                   85                  90                  95
   Pro Ala Leu Ser Leu Asp Ser Arg Glu Arg Glu Lys Thr Ser Thr Phe
               100                 105                 110
   Ala Arg Val Gly Ser Thr Ile Gly Ala Asn Leu Val Gly Val Val Ile
           115                 120                 125
   Thr Pro Ile Ile Leu Phe Phe Ser Ala Ser Lys Ala Asn Pro Asn Gly
       130                 135                 140
   Asp Lys Gln Gly Trp Phe Phe Phe Ala Leu Ile Val Ala Ile Val Gly
   145                 150                 155                 160
   Ile Leu Thr Ser Ile Thr Val Gly Leu Gly Thr His Glu Val Lys Ser
                   165                 170                 175
   Ala Leu Arg Glu Ser Asn Glu Lys Thr Thr Leu Lys Gln Val Phe Lys
               180                 185                 190
   Val Leu Gly Gln Asn Asp Gln Leu Leu Trp Leu Ala Phe Ala Tyr Trp
           195                 200                 205
   Phe Tyr Gly Leu Gly Ile Asn Thr Leu Asn Ala Leu Gln Leu Tyr Tyr
       210                 215                 220
   Phe Ser Tyr Ile Leu Gly Asp Ala Arg Gly Tyr Ser Leu Leu Tyr Thr
   225                 230                 235                 240
   Ile Asn Thr Phe Val Gly Leu Ile Ser Ala Ser Phe Phe Pro Ser Leu
                   245                 250                 255
   Ala Lys Lys Phe Asn Arg Asn Arg Leu Phe Tyr Ala Cys Ile Ala Val
               260                 265                 270
   Met Leu Leu Gly Ile Gly Val Phe Ser Val Ala Ser Gly Ser Leu Ala
           275                 280                 285
   Leu Ser Leu Val Gly Ala Glu Phe Phe Phe Ile Pro Gln Pro Leu Ala
       290                 295                 300
   Phe Leu Val Val Leu Met Ile Ile Ser Asp Ala Val Glu Tyr Gly Gln
   305                 310                 315                 320
   Leu Lys Thr Gly His Arg Asp Glu Ala Leu Thr Leu Ser Val Arg Pro
                   325                 330                 335
   Leu Val Asp Lys Leu Gly Gly Ala Leu Ser Asn Trp Phe Val Ser Leu
               340                 345                 350
   Ile Ala Leu Thr Ala Gly Met Thr Thr Gly Ala Thr Ala Ser Thr Ile
           355                 360                 365
   Thr Ala His Gly Gln Met Val Phe Lys Leu Ala Met Phe Ala Leu Pro
       370                 375                 380
   Ala Val Met Leu Leu Ile Ala Val Ser Ile Phe Ala Lys Lys Val Phe
   385                 390                 395                 400
   Leu Thr Glu Glu Lys His Ala Glu Ile Val Asp Gln Leu Glu Thr Gln
                   405                 410                 415
   Phe Ser Gln Ser His Ala Gln Lys Pro Ala Gln Ala Glu Ser Phe Thr
               420                 425                 430
   Leu Ala Ser Pro Val Ser Gly Gln Leu Met Asn Leu Asp Met Val Asp
           435                 440                 445
   Asp Pro Val Phe Ala Asp Lys Lys Leu Gly Asp Gly Phe Ala Leu Val
       450                 455                 460
   Pro Ala Asp Gly Lys Val Tyr Ala Pro Phe Ala Gly Thr Val Arg Gln
   465                 470                 475                 480
   Leu Ala Lys Thr Arg His Ser Ile Val Leu Glu Asn Glu His Gly Val
                   485                 490                 495
   Leu Val Leu Ile His Leu Gly Leu Gly Thr Val Lys Leu Asn Gly Thr
               500                 505                 510
   Gly Phe Val Ser Tyr Val Glu Glu Gly Ser Gln Val Glu Ala Gly Gln
           515                 520                 525
   Gln Ile Leu Glu Phe Trp Asp Pro Ala Ile Lys Gln Ala Lys Leu Asp
       530                 535                 540
   Asp Thr Val Ile Val Thr Val Ile Asn Ser Glu Thr Phe Ala Asn Ser
   545                 550                 555                 560
   Gln Met Leu Leu Pro Ile Gly His Ser Val Gln Ala Leu Asp Asp Val
                   565                 570                 575
   Phe Lys Leu Glu Gly Lys Asn
   580
<210> 3
   <211> 880
   <212> PRT
   <213> Lactobacillus bulgaricus
<400> 3
   Met Ser Asn Lys Leu Val Lys Glu Lys Arg Val Asp Gln Ala Asp Leu
   1 5 10 15
   Ala Trp Leu Thr Asp Pro Glu Val Tyr Glu Val Asn Thr Ile Pro Pro
   20 25 30
   His Ser Asp His Glu Ser Phe Gln Ser Gln Glu Glu Leu Glu Glu Gly
   35 40 45
   Lys Ser Ser Leu Val Gln Ser Leu Asp Gly Asp Trp Leu Ile Asp Tyr
   50 55 60
   Ala Glu Asn Gly Gln Gly Pro Val Asn Phe Tyr Ala Glu Asp Phe Asp
   65 70 75 80
   Asp Ser Asn Phe Lys Ser Val Lys Val Pro Gly Asn Leu Glu Leu Gln
   85 90 95
   Gly Phe Gly Gln Pro Gln Tyr Val Asn Val Gln Tyr Pro Trp Asp Gly
   100 105 110
   Ser Glu Glu Ile Phe Pro Pro Gln Ile Pro Ser Lys Asn Pro Leu Ala
   115 120 125
   Ser Tyr Val Arg Tyr Phe Asp Leu Asp Glu Ala Phe Trp Asp Lys Glu
   130 135 140
   Val Ser Leu Lys Phe Asp Gly Ala Ala Thr Ala Ile Tyr Val Trp Leu
   145 150 155 160
   Asn Gly His Phe Val Gly Tyr Gly Glu Asp Ser Phe Thr Pro Ser Glu
   165 170 175
   Phe Met Val Thr Lys Phe Leu Lys Lys Glu Asn Asn Arg Leu Ala Val
   180 185 190
   Ala Leu Tyr Lys Tyr Ser Ser Ala Ser Trp Leu Glu Asp Gln Asp Phe
   195 200 205
   Trp Arg Met Ser Gly Leu Phe Arg Ser Val Thr Leu Gln Ala Lys Pro
   210 215 220
   Arg Leu His Leu Glu Asp Leu Lys Leu Thr Ala Ser Leu Thr Asp Asn
   225 230 235 240
   Tyr Gln Lys Gly Lys Leu Glu Val Glu Ala Asn Ile Ala Tyr Arg Leu
   245 250 255
   Pro Asn Ala Ser Phe Lys Leu Glu Val Arg Asp Ser Glu Gly Asp Leu
   260 265 270
   Val Ala Glu Lys Leu Gly Pro Ile Arg Ser Glu Gln Leu Glu Phe Thr
   275 280 285
   Leu Ala Asp Leu Pro Val Ala Ala Trp Ser Ala Glu Lys Pro Asn Leu
   290 295 300
   Tyr Gln Val Arg Leu Tyr Leu Tyr Gln Ala Gly Ser Leu Leu Glu Val
   305 310 315 320
   Ser Arg Gln Glu Val Gly Phe Arg Asn Phe Glu Leu Lys Asp Gly Ile
   325 330 335
   Met Tyr Leu Asn Gly Gln Arg Ile Val Phe Lys Gly Ala Asn Arg His
   340 345 350
   Glu Phe Asp Ser Lys Leu Gly Arg Ala Ile Thr Glu Glu Asp Met Ile
   355 360 365
   Trp Asp Ile Lys Thr Met Lys Arg Ser Asn Ile Asn Ala Val Arg Cys
   370 375 380
   Ser His Tyr Pro Asn Gln Ser Leu Phe Tyr Arg Leu Cys Asp Lys Tyr
   385 390 395 400
   Gly Leu Tyr Val Ile Asp Glu Ala Asn Leu Glu Ser His Gly Thr Trp
   405 410 415
   Glu Lys Val Gly Gly His Glu Asp Pro Ser Phe Asn Val Pro Gly Asp
   420 425 430
   Asp Gln His Trp Leu Gly Ala Ser Leu Ser Arg Val Lys Asn Met Met
   435 440 445
   Ala Arg Asp Lys Asn His Ala Ser Ile Leu Ile Trp Ser Leu Gly Asn
   450 455 460
   Glu Ser Tyr Ala Gly Thr Val Phe Ala Gln Met Ala Asp Tyr Val Arg
   465 470 475 480
   Lys Ala Asp Pro Thr Arg Val Gln His Tyr Glu Gly Val Thr His Asn
   485 490 495
   Arg Lys Phe Asp Asp Ala Thr Gln Ile Glu Ser Arg Met Tyr Ala Pro
   500 505 510
   Ala Lys Val Ile Glu Glu Tyr Leu Thr Asn Lys Pro Ala Lys Pro Phe
   515 520 525
   Ile Ser Val Glu Tyr Ala His Ala Met Gly Asn Ser Val Gly Asp Leu
   530 535 540
   Ala Ala Tyr Thr Ala Leu Glu Lys Tyr Pro His Tyr Gln Gly Gly Phe
   545 550 555 560
   Ile Trp Asp Trp Ile Asp Gln Gly Leu Glu Lys Asp Gly His Leu Leu
   565 570 575
   Tyr Gly Gly Asp Phe Asp Asp Arg Pro Thr Asp Tyr Glu Phe Cys Gly
   580 585 590
   Asn Gly Leu Val Phe Ala Asp Arg Thr Glu Ser Pro Lys Leu Ala Asn
           595                 600                 605
   Val Lys Ala Leu Tyr Ala Asn Leu Lys Leu Glu Val Lys Asp Gly Gln
       610                 615                 620
   Leu Phe Leu Lys Asn Asp Asn Leu Phe Thr Asn Ser Ser Ser Tyr Tyr
   625                 630                 635                 640
   Phe Leu Thr Ser Leu Leu Val Asp Gly Lys Leu Thr Tyr Gln Ser Arg
                   645                 650                 655
   Pro Leu Thr Phe Gly Leu Glu Pro Gly Glu Ser Gly Thr Phe Ala Leu
               660                 665                 670
   Pro Trp Pro Glu Val Ala Asp Glu Lys Gly Glu Val Val Tyr Arg Val
           675                 680                 685
   Thr Ala His Leu Lys Glu Asp Leu Pro Trp Ala Asp Glu Gly Phe Thr
       690                 695                 700
   Val Ala Glu Ala Glu Glu Val Ala Gln Lys Leu Pro Glu Phe Lys Pro
   705                 710                 715                 720
   Glu Gly Arg Pro Asp Leu Val Asp Ser Asp Tyr Asn Leu Gly Leu Lys
                   725                 730                 735
   Gly Asn Asn Phe Gln Ile Leu Phe Ser Lys Val Lys Gly Trp Pro Val
               740                 745                 750
   Ser Leu Lys Tyr Ala Gly Arg Glu Tyr Leu Lys Arg Leu Pro Glu Phe
           755                 760                 765
   Thr Phe Trp Arg Ala Leu Thr Asp Asn Asp Arg Gly Ala Gly Tyr Gly
       770                 775                 780
   Tyr Asp Leu Ala Arg Trp Glu Asn Ala Gly Lys Tyr Ala Arg Leu Lys
   785                 790                 795                 800
   Asp Ile Ser Cys Glu Val Lys Glu Asp Ser Val Leu Val Lys Thr Ala
                   805                 810                 815
   Phe Thr Leu Pro Val Ala Leu Lys Gly Asp Leu Thr Val Thr Tyr Glu
               820                 825                 830
   Val Asp Gly Arg Gly Lys Ile Ala Val Thr Ala Asp Phe Pro Gly Ala
           835                 840                 845
   Glu Glu Ala Gly Leu Leu Pro Ala Phe Gly Leu Asn Leu Ala Leu Pro
       850                 855                 860
   Lys Glu Leu Thr Asp Tyr Arg Tyr Tyr Gly Leu Gly Pro Asn Glu Ser
   865                 870                 875                 880

## Description

Ces souches et ferments peuvent être utilisés pour l'obtention de produits laitiers fermentés à partir de lait additionné de glucose.

La présente Invention est relative à de nouveaux variants de *bulgaricus* et à leur utilisation pour la préparation de produits laitiers fermentés.

Les yoghourts sont traditionnellement obtenus par fermentation du lait avec une association de *Streptococcus* thermophilus et *Lactobacillus bulgaricus*. Au cours de la fermentation qui est effectuée à une température d'environ 40 à 45°C, ces bactéries utilisent principalement le lactose comme substrat énergétique, et produisent de l'acide lactique qui entraîne la coagulation du lait ; lorsque le pH atteint une valeur d'environ 4,8 à 4,5, on met fin à cette étape de fermentation (également dénommée « acidification ») en refroidissant le produit. Celui-ci est ensuite maintenu au froid pendant la suite du processus de fabrication et de conditionnement, et jusqu'à sa consommation.

Cependant, le refroidissement ne stoppe pas complètement la fermentation lactique ; même lorsque le produit est maintenu à 4°C, on observe une augmentation progressive de son acidité au cours du temps.

Ce phénomène, connu sous le nom de post-acidification, est responsable d'une dégradation des qualités organoleptiques du produit pendant sa conservation.

La post-acidification résulte essentiellement de l'utilisation par les bactéries, et principalement par *L. bulgaricus,* du lactose restant dans le produit à l'issue de l'étape d'acidification contrôlée. Pour l'éviter, il a été proposé d'utiliser des souches de *L. bulgaricus* ne fermentant pas, ou très peu, le lactose.

Une des enzymes essentielles pour la fermentation du lactose est la β-galactosidase, qui hydrolyse le lactose en glucose et galactose. Il a donc été proposé, pour obtenir des souches non-postacidifiantes de *L. bulgaricus,* de produire des mutants artificiels, ou de sélectionner des mutants naturels, chez lesquels l'activité de cette enzyme était affectée.

Par exemple, le Brevet EP 402 450 au nom de GENENCOR décrit l'obtention, par mutagénèse localisée du gène de la β-galactosidase, de mutants conditionnels de *L. bulgaricus,* chez lesquels la β-galactosidase qui est active lors de la fermentation à 40°C, perd son activité à la température ou au pH correspondant aux conditions de conservation des produits laitiers fermentés.

La Demande JP 90053437 décrit l'obtention d'un mutant artificiel de *L. bulgaricus* ayant complètement perdu la capacité de fermenter le lactose, et la sélection d'un mutant naturel, à capacité réduite de fermentation du lactose ; ces mutants sont néanmoins capables, l'un comme l'autre, de se développer et d'acidifier normalement en présence de *S. thermophilus,* à condition que le milieu soit supplémenté en glucose. Les sous-cultures de ces mutants conservent leurs caractéristiques d'acidification, dans du lait dépourvu de glucose, après 10 repiquages.

Le Brevet EP 0518 096, au nom de la SOCIÉTÉ DES PRODUITS NESTLÉ, propose d'utiliser pour la fabrication de yoghourt, des mutants faiblement postacidifiants de *Lactobacillus bulgaricus* préalablement sélectionnés sur le critère de la délétion d'un fragment du gène de la β-galactosidase. Le criblage et la caractérisation de ces mutants sont facilités, du fait que la présence de cette délétion peut être facilement vérifiés sur des profils de restriction. En outre, les délétions sont connues pour être des mutations irréversibles, ce qui permet d'obtenir facilement des souches mutantes stables à partir de la souche mère. Le Brevet EP 0518 096 décrit 2 types de mutants faiblement postacidifiants sélectionnés de la sorte. Les premiers ont une délétion touchant uniquement le gène de la β-galactosidase ; lorsqu'ils sont associés à *S. thermophilus* et cultivés sur du lait, ils présentent, même en l'absence d'ajout de glucose, des propriétés de croissance et d'acidification comparables à celles de la souche sauvage dont ils sont issus. Les seconds présentent une délétion plus importante, s'étendant sur au moins 1kb en aval du gène de la β-galactosidase ; lorsqu'ils sont associés à *S. thermophilus* ils croissent plus lentement et acidifient beaucoup moins que la souche sauvage dont ils sont issus ; l'ajout de glucose au milieu de culture n'a que peu d'influence sur leurs propriétés d'acidification et de post-acidification.

Les mutants naturels chez lesquels la β-galactosidase est inactive sont beaucoup plus difficiles à sélectionner et à maintenir en cultures pures dans le cas de mutations ponctuelles que dans le cas de mutants de délétion ; ceci s'explique par la probabilité plus faible qu'une mutation ponctuelle produise une protéine inactive, par la plus grande difficulté pour localiser et caractériser les mutations ponctuelles par des profils de restriction, et par le taux de réversion très important.

La Demanderesse a maintenant trouvé d'autres mutants naturels de *L. bulgaricus,* ne portant pas de délétion dans le gène codant pour la β-galactosidase, et présentant des caractéristiques technologiques avantageuses. Dans le cadre de la présente invention, un mutant non-sens, incapable d'assimiler le lactose, a été isolé à partir d'une culture d'un *L. bulgaricus* sauvage. Associé à *S. thermophilus,* en culture sur du lait, il croît et acidifie beaucoup plus lentement que la souche sauvage dont il est issu. En revanche, sa croissance et son acidification sont quasi-normales lorsque le lait est supplémenté en glucose.

La présente Invention a pour objet une souche mutante de *L. bulgaricus* dépourvue d'activité β-galactosidase, caractérisée en ce que ledit manque d'activité β-galactosidase résulte d'une une mutation introduisant un codon non-sens dans la séquence codant pour la β-galactosidase.

Une souche de *L. bulgaricus* conforme à l'invention a été déposée selon le Traité de Budapest le 14 janvier 1998, auprès de la CNCM (Collection Nationale de Cultures de Microorganismes) tenue par l'Institut Pasteur, 25 rue du Docteur Roux, à Paris, sous le numéro I-1968.

Cette souche présente les caractéristiques morphologiques et biochimiques suivantes :
- Morphologie : Microorganisme Gram-positif, bacilles fins, pléomorphes, asporogènes, isolés ou en courtes chaînes, immobiles.
- Métabolisme : homofermentaire, catalase (-).
- Fermentation des sucres : D-glucose (+), D-fructose (+), D-mannose (+), esculine (+).

Les Inventeurs ont séquence l'opéron lactose chez le mutant I-1968. La séquence correspondante est représentée dans la liste de séquences en annexe sous le numéro SEQ ID NO:1. Les séquences des produits de traduction (perméase et β-galactosidase) sont respectivement représentées sous les numéros SEQ ID NO:2 et SEQ ID NO:3

L'analyse de cette séquence fait apparaître deux mutations ponctuelles : l'une, au niveau du gène de la perméase (position 122 de la séquence SEQ ID NO:1), induit un changement d'acide aminé (Lys → Asn) ; l'autre, au niveau du gène de la β-galactosidase (position 4519 de la séquence SEQ ID NO:1), introduit un codon de terminaison. Bien que conservant ses sites actifs (positions 464 et 531), la β-galactosidase produite par ce mutant est inactive. Les Inventeurs ont en outre constaté que cette mutation restait stable après plusieurs séries de repiquages sur un milieu de culture contenant du glucose. En revanche, sur un milieu de culture en absence de glucose, cette mutation non-sens réverte très rapidement à un taux d'environ 10⁻⁶.

La présente invention englobe également des souches mutantes incapables d'assimiler le lactose, dérivées de la souche I-1968. De telles souches peuvent par exemple être obtenues en induisant par mutagénèse dirigée, d'autres mutations dans l'opéron lactose de la souche I-1968.

La présente Invention a également pour objet un ferment lactique, en particulier un ferment du yoghourt, caractérisé en ce qu'il comprend au moins une souche de *L. bulgaricus* conforme à l'Invention telle que définie ci-dessus, de préférence associée à au moins une souche de *S. thermophilus*.

Pour l'obtention d'un ferment conforme à l'invention, on peut utiliser n'importe quelle souche de S. thermophilus convenant pour la fabrication de yoghourt ; le choix d'une ou plusieurs souches de *S. thermophilus* peut être effectué en fonction des caractéristiques additionnelles que l'on souhaite éventuellement conférer au produit fini.

A titre d'exemple de souches de *S. thermophilus* pouvant être utilisées en association avec une souche de *L. bulgaricus* conforme à l'invention, on peut citer les souches suivantes, déposées auprès de la CNCM (Collection Nationale de Cultures de Microorganismes) tenue par l'Institut Pasteur, 25 rue du Docteur Roux, à Paris :
- la souche déposée le 25 août 1994, sous le numéro I-1470, et la souche déposée le 23 août 1995, sous le numéro I-1620; ces 2 souches sont décrites dans la Demande Européenne publiée sous le numéro 96/06924 ;
- les souches déposées le 30 décembre 1994, sous les numéros I-1520 et I-1521 ; ces 2 souches sont décrites dans la Demande Internationale PCT WO 96/20607 :

- la souche déposée le 24 octobre 1995 sous le numéro I-1630 ; les caractéristiques de cette souche sont décrites dans la Demande Internationale PCT WO 96/01701.

Ces souches peuvent être associées entre elles, ou avec une ou plusieurs autres souches industrielles de *S. thermophilus*.

La ou les souche(s) de *S. thermophilus* sont associées avec la ou les souche(s) de *L. bulgaricus* conformes à l'invention de la même manière et dans les mêmes proportions que dans les ferments du yoghourt traditionnels ; la population de bactéries *L. bulgaricus* conformes à l'invention peut par exemple représenter entre 10 et 90%, de préférence entre 20 et 50%, de la population bactérienne totale.

La présente Invention a également pour objet un procédé de préparation d'un produit laitier fermenté caractérisé en ce qu'il comprend une étape au cours de laquelle on fermente du lait à l'aide d'un ferment comprenant au moins une souche de *L. bulgaricus* conforme à l'Invention, en présence d'au moins un sucre assimilable par ladite souche ; il peut s'agir notamment du fructose, du mannose, et de préférence, du glucose. Avantageusement ledit produit laitier fermenté est un yoghourt.

Le procédé conforme à l'Invention est similaire aux procédés traditionnels de préparation du yoghourt en ce qui concerne les principales modalités de mise en oeuvre de l'étape d'acidification contrôlée ; en particulier cette acidification est effectuée à une température comprise entre 20 et 45°C, et de préférence entre 30 et 45°C, et en « batch », c'est à dire en une seule étape et en utilisant une seule cuve de fermentation.

La durée de cette étape d'acidification contrôlée est généralement de l'ordre de 6 à 24 heures, et de préférence de l'ordre de 6 à 16 heures ; elle est donc plus longue que dans le cas des procédés classiques de préparation de yoghourt (où elle est de 3 à 5 heures à 44°C). En effet, les souches de *L. bulgaricus* conformes à l'Invention, même associées à *S. thermophilus,* croissent et acidifient beaucoup plus lentement que les souches sauvages.

En outre, la vitesse de croissance et d'acidification des souches de *L. bulgaricus* conformes à l'invention varie très significativement en fonction de la quantité de glucose ajoutée au lait. Cette propriété permet de contrôler leur croissance et leur acidification, par simple addition de la quantité souhaitée de glucose en début de fermentation.

Les Inventeurs ont en outre observé que, lors de la mise en oeuvre de souches de *L. bulgaricus* ou de ferments conformes à l'Invention, l'acidification ralentit considérablement lorsque le pH atteint la zone de 4,8 à 4,5, (qui correspond à la zone de pH où l'on arrête l'acidification dans le cas d'un procédé traditionnel), et se stabilise, même si l'on maintient le lait à température de fermentation, à un pH plancher. La valeur de ce pH plancher dépend essentiellement de la quantité de glucose ajoutée.

Cette propriété permet de réduire, voire d'éliminer la phase de refroidissement utilisée dans les procédés traditionnels de fabrication du yoghourt pour stopper la fermentation. Elle supprime en outre la nécessité de mesurer le pH pour déterminer le moment optimal d'arrêt de la fermentation ; pour un ferment et une quantité de glucose ajouté déterminés, il est possible, sans risque de sur-acidification, d'arrêter la fermentation au bout d'une durée donnée, calculée en fonction du temps nécessaire pour atteindre le pH plancher. Ceci permet de mieux contrôler la régularité du pH final et la texture du produit en fin de fermentation.

Avantageusement, pour la mise en oeuvre du procédé conforme à l'invention, et selon le degré d'acidification auquel on souhaite parvenir, la quantité de glucose ajoutée au lait préalablement à la fermentation est comprise entre 0,5 et 10 g/l, de préférence entre 0,5 et 5 g/l.

Le produit fermenté obtenu de la sorte peut être conservé plusieurs heures à une température proche de la température de fermentation, sans baisse du pH, ce qui permet de supprimer les installations de stockage intermédiaire au froid et d'augmenter la capacité des cuves de fermentation.

La mise en oeuvre du procédé conforme à l'Invention permet de réduire la postacidifïcation dans les produits fermentés au cours de leur conservation à plus long terme. Le degré de postacidification peut varier selon la composition du ferment et la quantité de glucose utilisée. Cependant la postacidification est toujours nettement inférieure à celle observée dans le cas des yaourts obtenus avec les ferments et les procédés traditionnels.

Par exemple, des expérimentations effectuées par les Inventeurs ont montré que dans les mêmes conditions de conservation (28 jours de conservation à 10°C), le ΔpH (différence entre le pH à J0 et le pH à J28) était compris entre 0,05 et 0,4 dans le cas des produits obtenus à l'aide d'un ferment conforme à l'invention, alors qu'il était toujours supérieur à 0,7 dans le cas de ferments témoins dans lesquels la souche de *L. bulgaricus* conforme à l'invention était remplacée par une souche sauvage.

Cette faible post-acidification s'accompagne d'une bonne survie des souches du ferment ; la population de *L. bulgaricus* en fin de conservation, dans le produit fermenté obtenu conformément à l'invention n'est que légèrement inférieure à celle du produit témoin.

La présente invention a également pour objet les produits laitiers fermentés susceptibles d'être obtenus en mettant en oeuvre un procédé conforme à l'invention.

Ces produits peuvent se conserver pendant plus longtemps et à température plus élevées que les produits obtenus par les procédés traditionnels, et possèdent des caractéristiques organoleptiques qui restent stables au cours de la conservation.

### EXEMPLE 1 : DOSAGE BIOCHIMIQUE DE L'ACTIVITE BETA-GALACTOSIDASE D'UN MUTANT CONFORME A L'INVENTION.

L'activité β-galactosidase de la souche I-1968 a été comparée à celle de la souche sauvage de *L. bulgaricus* (dénommée ci-après LbS) dont elle est issue.

On cultive les bactéries pendant une nuit sur milieu MRS agar (MERCK) à 37°C, en jarre d'anaérobiose (MERCK) en présence d'un fixateur d'oxygène (AnaérocultA®, MERCK).

Une öse de 10 microlitres (NUNC) de bactéries est resuspendue dans 1 millilitre d'eau stérile. Les bactéries sont lysées par 2 cycles d'agitation forte, 20 secondes à 5000 rotations par minute en présence de microbilles de verre (0,5 mm de diamètre, BIOSPEC PRODUCTS), puis rajout de 0,15 ml de chloroforme. On agite l'ensemble pendant 30 minutes à 37°C, puis on complète à 2 ml avec de l'eau stérile à 4°C. L'activité bêta-galactosidase est alors mesurée : à partir de 0,2 ml de la suspension cellulaire on rajoute 1,2 ml de tampon NaH₂PO₄ 0,067M ; pH6,8 ; 0,05 ml de L-cystéine (SIGMA) à t0 0,05 ml de *O*-nitrophényl-bêta-D-galactopyranoside (SIGMA). La réaction enzymatique est arrêtée après 0, 2, 5 ou 10 min. par 1 ml de tampon Na₂CO₃ 10%, et on effectue une mesure de la DO à 400 nanomètres sur le surnageant, après centrifugation du milieu réactionnel.

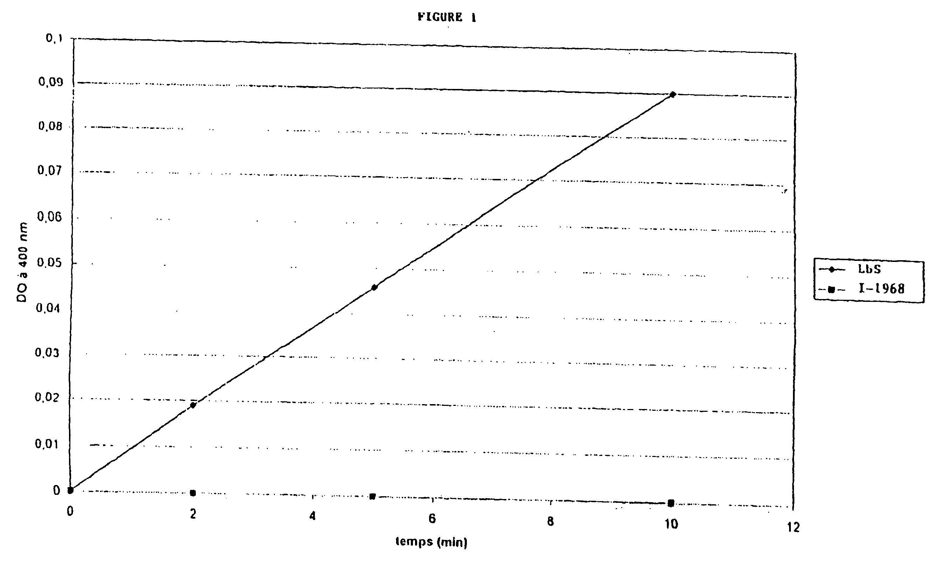

Les activités galactosidase de la souche-mère LbS et du mutant I-1968 conforme à l'Invention mesurées en fonction du temps sont représentées sur la Figure 1.

Ces résultats montrent que la β-galactosidase est totalement inactive chez le mutant conforme à l'Invention.

### EXEMPLE 2 : STABILITÉ DU MUTANT I-1968 DE L. BULGARICUS

La stabilité du mutant I-1968 a été testée dans des milieux contenant comme sources de carbone soit un mélange de glucose et de lactose, soit du lactose seulement.
Une culture de I-1968 obtenue sur milieu MRS contenant du glucose est mise en sous-culture sur du lait stérilisé additionné d'autolysat de levure (2g/l), et supplémenté ou non avec du glucose (20g/l). Lorsqu'un pH de 5,2 (coagulation du lait) est atteint, on prélève des échantillons de chaque sous-culture, sur lesquels on analyse la capacité des bactéries à fermenter les sucres, ainsi que la présence d'activité β-galactosidase (test sur plaque X-gal : colonies blanches = β-galactosidase moins ; colonies bleues = β-galactosidase plus).
Les résultats sont illustrés par le Tableau I ci-dessous.

**TABLEAU I**

| Milieu | Lait + glucose (20g/l) | Lait |
|---|---|---|
| Temps pour atteindre pH 5.2 | 6h00 | 20h00 |
| Fermentation des sucres | glucose, fructose, mannose | lactose, glucose, fructose, mannose |
| Test sur plaque X-gal | 100% Colonies blanches | 20% colonies blanches 80% colonies bleues |

Ces résultats montrent qu'en présence de glucose, la souche I-1968 ne réverte pas vers une souche capable d'utiliser le lactose. En revanche dans un milieu contenant du lactose comme seule source de carbone, on observe une réversion rapide de la souche I-1968 vers l'état original..

### EXEMPLE 3 : PROPRIÉTÉS D'ACIDIFICATION, DE POSTACIDIFICATION ET DE SURVIE DU VARIANT I-1968 DE L. BULGARICUS EN SYMBIOSE AVEC S. THERMOPHILUS : CAS D'UN PROCÉDÉ DE FABRICATION D'UN YOGHOURT FERME (FERMENTATION EN ÉTUVE VENTILÉE)

On prépare des ferments de yoghourt associant la souche I-1968 conforme à l'Invention avec différentes souches industrielles de *S. thermophilus* (les souches de *S. thermophilus* utilisées sont dénommées ci-après ST1, ST2 et ST3).

A titre de comparaison, on prépare des ferments associant la souche-mère LbS, et les mêmes souches de *S. thermophilus*.

Pour la préparation des ferments, les souches sont ensemencées séparément à 1% sur la composition suivante :
Composition pour 1 litre :
   135 g de poudre de lait écrémé
   2 g d'autolysat de levure
   920 ml d'eau distillée
   20 g de glucose (pour la souche I-1968 uniquement)
   Hydratation: 10 min
   Pasteurisation: 30 min à 95°C

Le lait est ensuite refroidi à 44°C et inoculé, puis incubé à 44°C jusqu'à obtention d'une acidité de 85°D (degrés Dornic) pour les streptocoques et de 80°D pour les lactobacilles.

Les cultures sont ensuite réunies pour obtenir un ferment constitué à 80% de *Streptococcus thermophilus* et à 20% de *Lactobacillus bulgaricus*.

Les ferments ainsi obtenus sont utilisés pour inoculer la préparation suivante :
Composition pour 1 litre :
   99% de lait
   0, 1, ou 2 g/l de glucose
   Hydratation: 10 min
   Pasteurisation: 10 min à 95°C

Le lait est ensuite refroidi à 44°C et inoculé à 1%.

Pour chaque expérimentation, la composition du ferment et la quantité de glucose ajoutée sont indiquées dans le Tableau II ci-dessous :

**TABLEAU II**

| **Expérience** | **Glucose g/l** | **Souches** | **Pourcentage** |
|---|---|---|---|
| 1 | 0 | ST 3 | 64 % |
| | | ST 2 | 16% |
| | | LbS | 20 % |
| 2 | 0 | ST 3 | 64 % |
| | | ST 2 | 16 % |
| | | I-1968 | 20 % |
| 3 | 1 | ST 3 | 64 % |
| | | ST 2 | 16 % |
| | | I-1968 | 20 % |
| 4 | 0 | ST1 | 80% |
| | | LbS | 20 % |
| 5 | 0 | ST 1 | 80 % |
| | | I-1968 | 20 % |
| 6 | 2 | ST 1 | 80 % |
| | | I-1968 | 20 % |

Après inoculation, le lait est réparti en ballons et incubé à une température de 44°C. Le profil d'acidification est suivi pendant l'incubation. Les produits sont décaillés à pH 4,6 par refroidissement en cellule froide (16 heures à 4°C).

Les produits sont ensuite soumis à un test de conservation à 10°C. Dans ce test, on mesure le pH et l'acidité Dornic après 1, 14, 21 et 28 jours de conservation.

Les résultats d'acidification (temps pour atteindre un pH de 4,6 et valeur du pH à 24h) sont présentés dans le tableau III ci-dessous :

**TABLEAU III**

| **Expérience** | Temps pour atteindre pH 4,6 (min) | Temps pour atteindre pH 4,5 (min) | pH à 24h |
|---|---|---|---|
| **1** | 215 | 236 | 3,67 |
| **2** | 550 | 778 | 4,33 |
| **3** | 416 | 507 | 4,26 |
| **4** | 225 | 241 | 3,67 |
| **5** | 660 | >1500 | 4,54 |
| **6** | 390 | 465 | 4,35 |

Les résultats du test de conservation à 10°C (suivi du pH et de l'acidité Dornic), et de la survie (populations de *S. thermophilus* et *L. bulgaricus*) à 28 jours sont présentés dans le tableau IV ci-dessous :

**TABLEAU IV**

| **Expérience** | Temps de stockage (jours) | pH | Acidité Dornic | *Streptococcus thermophilus* cellules/ml | *Lactobacillus bulgaricus* cellules/ml |
|---|---|---|---|---|---|
| **1** | 1 | 4,41 | 101 | 7,25E+08 | 3,35E+08 |
| **1** | 14 | 3,98 | 140 | ND | ND |
| **1** | 21 | 3,95 | 145 | ND | ND |
| **1** | 28 | 3,9 | 148 | 7,35E+08 | 3,30E+08 |
| **2** | 1 | 4,5 | 93 | 5,60E+08 | 2,90E+07 |
| **2** | 14 | 4,23 | 110 | ND | ND |
| **2** | 21 | 4,18 | 112 | ND | ND |
| **2** | 28 | 4,19 | 114 | 5,65E+08 | 1,87E+07 |
| **3** | 1 | 4,49 | 96 | 6,90E+08 | 7,45E+07 |
| **3** | 14 | 4,14 | 115 | ND | ND |
| **3** | 21 | 4,15 | 117 | ND | ND |
| **3** | 28 | 4,15 | 120 | 8.65E+08 | 6,30E+07 |
| **4** | 1 | 4,39 | 105 | 6,30E+07 | 4,40E+08 |
| **4** | 14 | 3,91 | 145 | ND | ND |
| **4** | 21 | 3,9 | 151 | ND | ND |
| **4** | 28 | 3,85 | 157 | 4,70E+08 | 6,40E+08 |
| **5** | 1 | 4,6 | 85 | 9,05E+08 | 6,70E+07 |
| **5** | 14 | 4,58 | 80 | ND | ND |
| **5** | 21 | 4,53 | 80 | ND | ND |
| **5** | 28 | 4,61 | 79 | 9,40E+08 | 7,00E+07 |
| **6** | 1 | 4,51 | 89 | 1,05E+09 | 1,96E+08 |
| **6** | 14 | 4,38 | 90 | ND | ND |
| **6** | 21 | 4,39 | 96 | ND | ND |
| **6** | 28 | 4,42 | 90 | 1,62E+09 | 1,91E+08 |
| ND : Non Déterminé | | | | | |

Ces résultats montrent que les yoghourts réalisés avec les symbioses associant la souche I-1968 à une ou deux souches de *S. thermophilus* présentent une postacidification extrêmement réduite par rapport aux mêmes symbioses avec la souche-mère LbS, tout en conservant une population abondante en fin de fermentation et une bonne survie pendant 28 jours à 10°C.

L'arrêt de l'acidification et le maintien du pH aux alentours de 4,6 à 4,5 pendant au moins 24 heures à 44°C permet dans le cadre de la fabrication de yoghourt brassé, de réduire, voire d'éliminer la phase de refroidissement en cuve qui est habituellement mise en oeuvre.

## Revendications

1. Souche mutante de *L. bulgaricus* dépourvue d'activité β-galactosidase, **caractérisée en ce que** ledit manque d'activité β-galactosidase résulte d'une mutation non-sens dans la séquence codant pour la β-galactosidase.

2. Souche mutante de *L. bulgaricus* selon la revendication 1, déposée le 14 janvier 1998, auprès de la CNCM sous le numéro I-1968.

3. Ferment lactique, **caractérisé en ce qu'**il comprend au moins une souche de *L. bulgaricus* selon la revendication 1 ou la revendication 2.

4. Ferment lactique selon la revendication 3 **caractérisé en ce que** ladite souche de *L. bulgaricus* est associée à au moins une souche de *S. thermophilus.*

5. Procédé de préparation d'un produit laitier fermenté, **caractérisé en ce qu'**il comprend une étape au cours de laquelle on fermente du lait à l'aide d'un ferment lactique comprenant au moins une souche de *L. bulgaricus* selon une quelconque des revendications 1 ou 2, en présence d'au moins un sucre assimilable par ladite souche.

6. Procédé selon la revendication 5, **caractérisé en ce que** ledit sucre assimilable est le glucose.

7. Procédé selon une quelconque des revendications 5 ou 6, **caractérisé en ce que** l'arrêt de la fermentation s'effectue sans refroidissement dudit produit laitier.

8. Produit laitier fermenté susceptible d'être obtenu par un procédé selon une quelconque des revendications 5 à 7.

9. Produit laitier fermenté selon la revendication 8, **caractérisé en ce que** ledit produit est un yoghourt.

## Patentansprüche

1. Mutantenstamm von *L. bulgaricus,* der an β-Galactosidase-Aktivität verarmt ist, **dadurch gekennzeichnet, dass** der genannte Mangel an β-Galactosidase-Aktivität aus einer Nichtsinn-Mutation in der Sequenz, die für die β-Galactosidase kodiert, resultiert.

2. Mutantenstamm von *L. bulgaricus* nach Anspruch 1, der am 19. Januar 1998 gemäß CNCM unter der Nummer 1-1968 hinterlegt wurde.

3. Milchferment, **dadurch gekennzeichnet, dass** es mindestens einen *L. bulgaricus*-Stamm nach Anspruch 1 oder Anspruch 2 umfasst.

4. Milchferment nach Anspruch 3, **dadurch gekennzeichnet, dass** der *L. bulgaricus*-Stamm mit mindestens einem *S. thermophilus*-Stamm kombiniert ist.

5. Verfahren zur Herstellung eines fermentierten Milchproduktes, **dadurch gekennzeichnet, dass** es eine Stufe umfasst, im Verlauf der man Milch mit Hilfe eines Milchferments, das mindestens einen *L. bulgaricus*-Stamm nach einem der Ansprüche 1 oder 2 umfasst, in Gegenwart mindestens eines durch den Stamm assimilierbaren Zuckers fermentiert.

6. Verfahren nach Anspruch 5, **dadurch gekennzeichnet, dass** der assimilierbare Zucker Glukose ist.

7. Verfahren nach einem der Ansprüche 5 oder 6, **dadurch gekennzeichnet, dass** das Anhalten der Fermentation ohne Abkühlen des Milchproduktes erfolgt.

8. Fermentiertes Milchprodukt, das durch ein Verfahren nach einem der Ansprüche 5 bis 7 erhältlich ist.

9. Fermentiertes Milchprodukt nach Anspruch 8, **dadurch gekennzeichnet, dass** das Produkt ein Joghurt ist.

## Claims

1. Mutant strain of *L. bulgaricus* devoid of β-galactosidase activity, **characterised in that** said lack of β-galactosidase activity results from a non-directional mutation in the sequence coding for β-galactosidase.

2. Mutant strain of *L. bulgaricus* according to Claim 1, filed on 14 January 1998 with the CNCM under the number 1-1968.

3. Lactic ferment, **characterised in that** it comprises at least one strain of *L. bulgaricus* according to claim 1 or claim 2.

4. Lactic ferment according to Claim 3, **characterised in that** the said strain of *L. bulgaricus* is associated with at least one strain of *S. thermophilus.*

5. Method of preparing a fermented milk product, **characterised in that** it comprises a step during which milk is fermented by means of a lactic ferment comprising at least one strain of *L. bulgaricus* according to either one of Claims 1 or 2, in the presence of at least one sugar which can be assimilated by the said strain.

6. Method according to Claim 5, **characterised in that** the said assimilatable sugar is glucose.

7. Method according to either one of Claims 5 or 6, **characterised in that** the fermentation is stopped without cooling of the said milk product.

8. Fermented milk product obtainableby amethod according to any one of Claims 5 to 7.

9. Fermented milk product according to Claim 8, **characterised in that** the said product is a yoghurt.
